# EUROPEAN PATENT APPLICATION

(11) **EP 2 835 129 A2**
(43) Date of publication of application: **11.02.2015**
(21) Application number: 14179362.0
(22) Date of filing: 31.07.2014
(51) Int. Cl.: A61K 8/02, B44C 1/17, A45D 33/38, A45D 40/30, A61Q 1/10

(54) **Transferable make-up member**

(30) Priority: 06.08.2013 KR 20130093068
(71) Applicant: JC Korea Corp., Gyeonggi-do (KR)
(72) Inventor: Kim, Dong Sung, Gyeonggi-do (KR); Kim, Hyun Surk, Gyeonggi-do (KR); Choi, Kyung Sik, Gyeonggi-do (KR); Park, Ju Young, Gyeonggi-do (KR); Choi, Jeong Rim, Gyeonggi-do (KR); Kim, Bo Mi, Seoul (KR)
(74) Representative: Jansen, Cornelis Marinus

(57) **Abstract**

The present disclosure relates to a transferable make-up member, and the transferable make-up member according to the present disclosure includes a design layer (110) including an ink and a vehicle, the design layer (110) having a design presented thereon to be transferred to a body (200), a release layer (120) provided on one surface of the design layer (110), the release layer (120) which is removed after the design layer is transferred to the body, and a protection film (130) provided on the other surface of the design layer (110), the protection film (130) which is removed before the design layer is transferred to the body (200).

## Description

### TECHNICAL FIELD

The present disclosure relates to a transferable make-up member.

### BACKGROUND

Generally, eyeliner serves to define the contours of the eyes by drawing a line on the eyelid and is used to make eyes look more defined, lipstick is used to apply desired colors to the lips, and a manicure is used to apply desired colors or designs to fingernails/toenails. A make-up member such as eyeliner, lipstick, and a manicure may not only allow the designs of eyes, lips, and fingernails/toenails to be in harmony with the shape of the face, but also may be used to change a look of a user.

Conventionally, a user has applied desired colors or designs on the body directly by defining the eyes using an eye pencil (see patent literature of related literatures below) or by applying a rouge or a liquid-type solution to lips or fingernails/toenails. However, because such colors or designs are applied to the body of which a majority of parts have a curved shape, applying desired colors or designs favorably is difficult. Moreover, in use of a manicure, an applied state of a manicure on the nails of both hands is not even depending on whether a user is right-handed or left-handed. Also, in use of eyeliner, make-up is applied with one eye closed, but keeping only one eye closed for a long time is difficult, and for this reason, an unskilled ordinary person has difficulty in defining the eyes himself/herself. Also, even if make-up is applied using eyeliner, lipstick and a manicure, an ordinary person, but not a skilled person such as a make-up artist, has difficulty in applying various designs delicately. For example, other than making eyes look more defined by drawing a line along the eye rims, it was difficult to create various lines around the eyes and implement various designs on fingernails/toenails. Accordingly, conventional eyeliners, lipsticks and manicures have disadvantages in that it takes much time to apply designs to the body, and even though make-up is applied for a long time, expressing as desired designs is difficult.

### [RELATED LITERATURES]

### Patent Literature

(Patent Literature 1) KR20-0270364 Y1

### SUMMARY

The present disclosure is designed to solve the problem of the related art, and according to one aspect of the present disclosure, there is provided a transferable make-up member that may transfer a design layer to the body to apply desired colors or designs to the body without a separate make-up member such as eyeliner, lipstick and a manicure.

A transferable make-up member according to exemplary embodiments of the present disclosure includes a design layer including an ink and a vehicle, the design layer having a design presented thereon to be transferred to a body, a release layer provided on one surface of the design layer, the release layer which is removed after the design layer is transferred to the body, a transfer inducing material coated between the design layer and the release layer or added to the design layer, the transfer inducing material including synthetic resin particles, and a protection film provided on the other surface of the design layer, the protection film which is removed before the design layer is transferred to the body.

Also, in the transferable make-up member according to exemplary embodiments of the present disclosure, the design layer has an adhesive substance added thereto.

Also, in the transferable make-up member according to exemplary embodiments of the present disclosure, the transferable make-up member further includes an adhesive layer formed between the design layer and the protection film.

Also, in the transferable make-up member according to exemplary embodiments of the present disclosure, the transferable make-up member is formed flat or in three dimensions to have a curve.

Also, in the transferable make-up member according to exemplary embodiments of the present disclosure, the design layer is transferred to a skin including eyes, lips, fingernails or toenails of the body.

Also, in the transferable make-up member according to exemplary embodiments of the present disclosure, the design layer is formed by silk screen printing, offset printing, mold printing, or gravure printing.

Also, in the transferable make-up member according to exemplary embodiments of the present disclosure, the synthetic resin particles include silica, glass, acrylic, or urethane.

Also, in the transferable make-up member according to exemplary embodiments of the present disclosure, the transfer inducing material is formed from an ultraviolet (UV) paint, a solvent-type paint or an aqueous paint, including the synthetic resin particles.

Also, in the transferable make-up member according to exemplary embodiments of the present disclosure, the release layer includes a handle formed to protrusively extend outward from one end.

Also, in the transferable make-up member according to exemplary embodiments of the present disclosure, the design layer is formed in a multilayered structure, and includes a base layer, and a first design layer and a second design layer respectively formed on both surfaces of the base layer, and the first design layer and the second design layer have designs presented thereon corresponding to each other.

Also, in the transferable make-up member according to exemplary embodiments of the present disclosure, the first design layer and the second design layer are disposed at corresponding locations to each other.

Also, in the transferable make-up member according to exemplary embodiments of the present disclosure, the design layer has light reflective particles added thereto, including glitter, bling, spangle, confetti, or pearl.

Also, in the transferable make-up member according to exemplary embodiments of the present disclosure, the release layer is formed of a polyethyleneterephthalate (PET) film or a polyethylene (PE) film coated with a silicone release agent or a fluoride release agent.

Also, in the transferable make-up member according to exemplary embodiments of the present disclosure, the release layer has an elastic layer formed from paper, polyethylene, ethylene vinyl acetate, polypropylene, latex, urethane, or foam.

Also, in the transferable make-up member according to exemplary embodiments of the present disclosure, the release layer is formed from paper, polyethylene, ethylene vinyl acetate, polypropylene, latex, urethane, or foam.

Also, in the transferable make-up member according to exemplary embodiments of the present disclosure, the release layer has a cutting line which is cut with respect to one point between both ends in a lengthwise direction.

Also, in the transferable make-up member according to exemplary embodiments of the present disclosure, the release layer includes an additional design layer having a design presented thereon corresponding to the design layer.

The features and advantages of the present disclosure will become more apparent from the following description with reference to the accompanying drawings.

Prior to the description, it should be understood that the terms or words used in the specification and the appended claims should not be construed as limited to general and dictionary meanings, but interpreted based on the meanings and concepts corresponding to technical aspects of the present disclosure on the basis of the principle that the inventor is allowed to define terms appropriately for the best explanation.

For the purpose of clarity and a concise description features are described herein as part of the same or separate embodiments. However, it will be appreciated that the scope of the invention may include embodiments having combinations of all or some of the features described.

### Effect of the Invention:

According to the present disclosure, by transferring the design layer to the body, there is an advantage that various colors or designs can be applied to the body accurately in a short time without a separate make-up member such as eyeliner, lipstick and a manicure.

Also, according to the present disclosure, by providing the transfer inducing material including the synthetic resin particles, there is an effect of transferring the design layer to the body effectively by printing the design layer on the release layer and separating the design layer from the release layer with ease.

Also, according to the present disclosure, by forming the elastic layer having relatively high elasticity in the release layer, there is an advantage that the transferable make-up member conforms to the curves of the body when transferring.

Also, according to the present disclosure, because the vehicle of the design layer has a hydrophobic property, the transferred design layer does not easily wash off with water, and as the vehicle is lipophilic, there is an effect of easily removing the transferred design layer with oil.

Also, according to the present disclosure, by forming the transferable make-up member in three dimensions to have a curve, there is an advantage that the transferable make-up member can be easily transferred to the body of which a majority of parts has a curved shape.

Also, according to the present disclosure, as the design layer includes the base layer and the first and second design layers having corresponding designs presented on both surfaces of the base layer, there is an effect of consistency between a design recognized by a user when transferring and a design seen by others after transferring.

Also, according to the present disclosure, there is an advantage that eye line and eye shadow can be simultaneously applied to the eyes of a user, and a difficult make-up such as gradation can be easily applied.

### BRIEF DESCRIPTION OF THE DRAWINGS

FIG. 1 is a bottom perspective view illustrating a transferable make-up member according to an exemplary embodiment of the present disclosure.
FIGS. 2 and 3 are cross-sectional views illustrating a transferable make-up member according to an exemplary embodiment of the present disclosure.
FIGS. 4a through 4c are plane views illustrating the transferable make-up member shown in FIG. 1.
FIG. 5 is a cross-sectional view illustrating a modified example of a transferable make-up member according to an exemplary embodiment of the present disclosure.
FIGS. 6 through 8 are perspective views illustrating a process of transferring a transferable make-up member according to an exemplary embodiment of the present disclosure.
FIG. 9 is a cross-sectional view illustrating a transferable make-up member according to another exemplary embodiment of the present disclosure.

### DETAILED DESCRIPTION OF EMBODIMENTS

The advantages, features and aspects of the present disclosure will become apparent from the following detailed description and preferred embodiments in conjunction with the accompanying drawings. In the assignment of reference numerals to components of each drawing in the specification, it should be noted that same components are allowed to have same numbers as possible although shown in other drawings. Also, the terms "first", "second", "one surface" and "the other surface" are used for the purpose of distinguishing one component from other component, and components are not limited by the terms. Hereinafter, in the description of the present disclosure, related known arts that would obscure the essence of the present disclosure are not described in detail but are omitted herein.

Hereinafter, the preferred embodiments of the present disclosure will be described in detail with reference to the accompanying drawings.

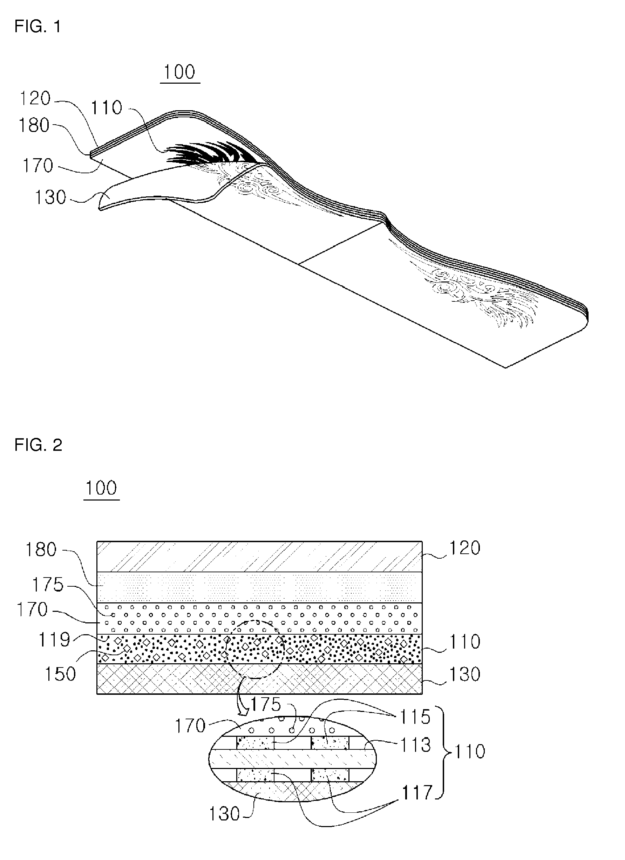

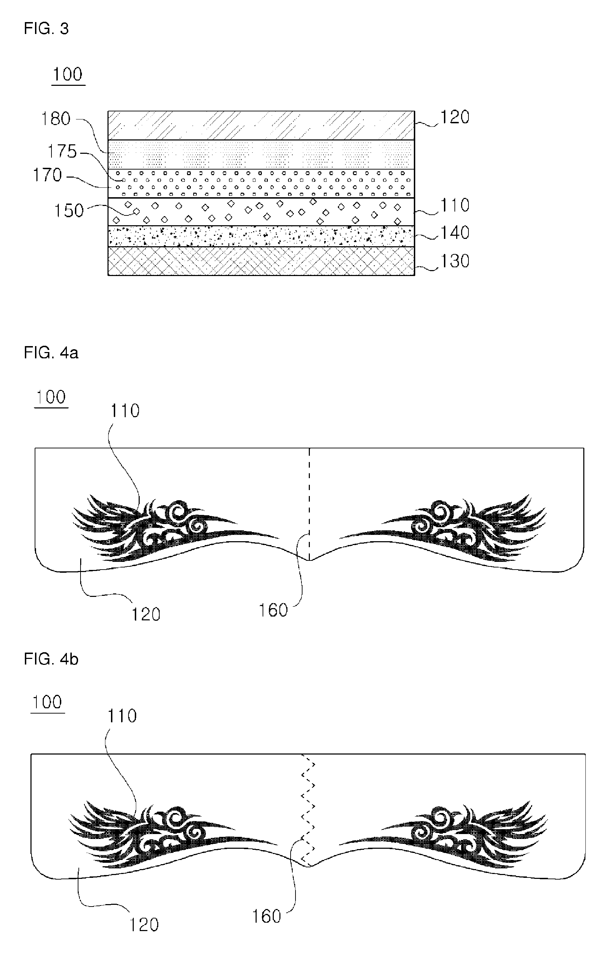

FIG. 1 is a bottom perspective view illustrating a transferable make-up member according to an exemplary embodiment of the present disclosure. FIGS. 2 and 3 are cross-sectional views illustrating a transferable make-up member according to an exemplary embodiment of the present disclosure. FIGS. 4a through 4c are plane views illustrating the transferable make-up member shown in FIG. 1.

As shown in FIGS. 1 through 4, a transferable make-up member 100 according to an exemplary embodiment of the present disclosure includes a design layer 110 which includes an ink and a vehicle and has a design presented thereon to be transferred to the body, a release layer 120 which is provided on one surface of the design layer 120 and is removed after the design layer 110 is transferred to the body 200, a transfer inducing material 170 which is coated between the design layer 110 and the release layer 120 or added to the design layer 110 and includes synthetic resin particles 175, and a protection film 130 which is provided on the other surface of the design layer 110 and is removed before the design layer 110 is transferred to the body 200.

The design layer 110 has a design presented thereon to be transferred to the body, and includes an ink and a vehicle. Here, the ink is used to impart colors or designs, for example, a pigment which is safe for a human body, that is, a substance defined in the Code of Federal Regulations (CFR) 21. Also, the vehicle serves to transport and attach the ink. In this instance, because the vehicle has a hydrophobic property, a waterproof effect may be produced so that the transferred design layer 110 does not easily wash off with water. Also, because the vehicle is lipophilic, the transferred design layer 110 can be easily removed with oil according to necessity. Meanwhile, using the design layer 110, various designs may be transferred to the body accurately. For example, eye line and eye shadow may be simultaneously applied to the eyes of a user, and a difficult make-up such as gradation may be easily applied. Also, after the design layer 110 is transferred to the body, an additional make-up may be applied.

Meanwhile, a method of forming the design layer 110 is not particularly limited, but the design layer 110 may be formed by silk screen printing, offset printing, mold printing, gravure printing, or other printing. In this instance, the design layer 110 may be formed in a multilayered structure by iteratively performing silk screen printing. That is, various colors and designs may be implemented by repeating silk screen printing plural times. However, when the design layer 110 is formed in a multilayered structure, a design seen in a direction toward one surface of the design layer 110 and a design seen in a direction toward the other surface (surface opposite to one surface) of the design layer 110 may differ. Accordingly, there is a concern that a design recognized by a user when transferring may be different from a design seen by others after transferring. However, as shown in the enlarged view of FIG. 2, the design layer 110 includes a base layer 113 and first and second design layers 115 and 117 to ease such concern. Specifically, the first design layer 115 and the second design layer 117 may be formed on both surfaces of the base layer 113. In this instance, the first design layer 115 and the second design layer 117 may have corresponding designs presented thereon, and may be disposed at corresponding locations. Accordingly, because a design (the first design layer 115) seen in a direction toward one surface of the design layer 110 and a design (the second design layer 117) seen in a direction toward the other surface of the design layer 110 are the same, a design recognized by a user when transferring may be consistent with a design seen by others after transferring. However, the expression "designs or locations of the first design layer 115 and the second design layer 117 correspond to each other" as used herein does not necessarily imply that they are mathematically the same, and is a concept encompassing a tolerance of a printing process, discoloration, and the like. Also, the scope of the present disclosure is not necessarily limited by "designs or locations of the first design layer 115 and the second design layer 117 correspond to each other". For example, even if the design layer 110 is formed in a multilayered structure, inconsistency between a design recognized by a user when transferring and a design seen by others after transferring may be prevented by keeping designs of each layer from overlapping while printing.

Additionally, the design layer 110 may have light reflective particles 150 added thereto, including glitter, bling, spangle, confetti, or pearl. Because the light reflective particles 150 have a luminous property, when added to the design layer 110, the light reflective particles 150 may realize a feeling as if they are jewels having a glittering property. In this instance, the light reflective particles 150 may be added to the design layer 110 through various methods such as scattering (free fall), thereby realizing a desired shape or size.

Also, as shown in FIG. 2, the design layer 110 may have an adhesive substance 119 added thereto. Here, the adhesive substance 119 serves to adhere the design layer 110 to the body, and because the adhesive substance 119 is a skin-friendly substance, is harmless to the body. When the adhesive substance 119 is added to the design layer 110, a separate adhesive layer is unnecessary. However, the adhesive substance 119 does not necessarily need to be added to the design layer 110, and as shown in FIG. 3, a separate adhesive layer 140 may be formed. Here, the adhesive layer 140 may be formed between the design layer 110 and the protection film 130. In this instance, the adhesive layer 140 serves to adhere the design layer 110 to the body and is skin-friendly and harmless to the body, similar to the adhesive substance 119.

The release layer 120 is removed after the design layer 110 is transferred to the body, and is provided on one surface of the design layer 110. Here, the release layer 120 may be formed of a polyethyleneterephthalate (PET) film or a polyethylene (PE) film coated with a silicone release agent or a fluoride release agent. The release layer 120 may have an elastic layer 180 including a synthetic or natural polymer structure such as paper, polyethylene, ethylene vinyl acetate, polypropylene, latex, urethane, or foam. Because the elastic layer 180 has relatively high elasticity, the elastic layer 180 may conform to the curves of the body during transferring. However, the release layer 120 and the elastic layer 180 do not necessarily need to be formed as separate layers, and the release layer 120 itself may be formed from paper, polyethylene, ethylene vinyl acetate, polypropylene, latex, urethane, or foam having relatively high elasticity.

Meanwhile, to separate the release layer 120 more easily, as shown in FIGS. 4a and 4b, a cutting line 160 cut with respect to one point between both ends in the lengthwise direction of the release layer 120 may be formed. In this instance, the cutting line 160 may extend perpendicularly to the lengthwise direction of the release layer 120, and may extend in a straight line (see FIG. 4a) or in a zigzag (see FIG. 4b). With the cutting line 160, a user may easily remove two release layers 120 disposed at both sides.

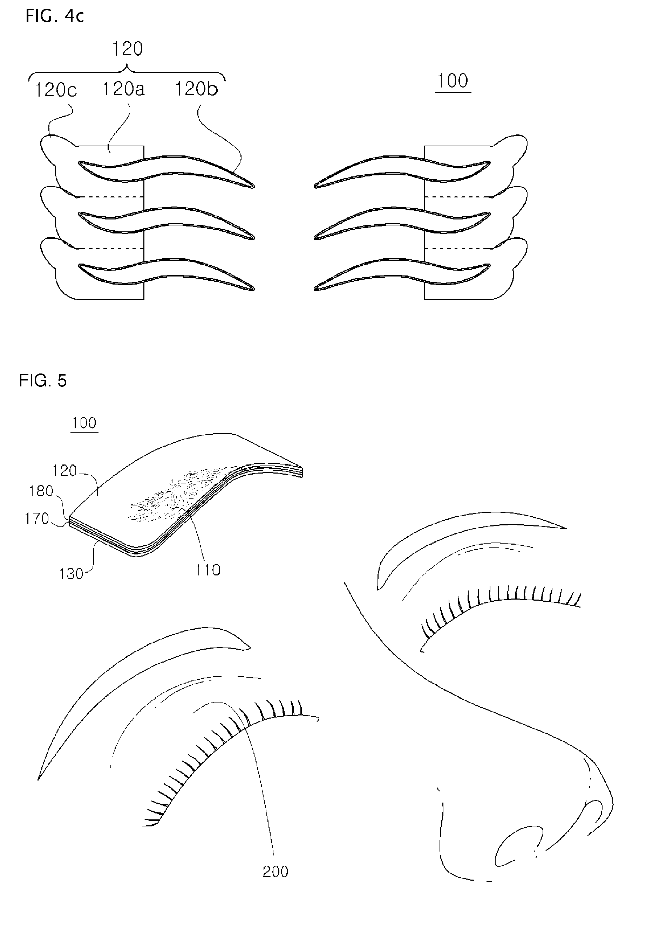

Also, as shown in FIG. 4c, the release layer 120 may have a handle 120c. Specifically, the release layer 120 may have an area at one end 120a larger than an area at the other end 120b, and the handle 120c may be formed to protrusively extend outward from one end 120a of the release layer 120. Accordingly, the user may hold the handle 120c and transfer the design layer 110 to a correct location of the body.

The transfer inducing material 170 (see FIGS. 1 through 3) may be coated between the design layer 110 and the release layer 120. Here, the transfer inducing material 170 may be formed from an ultraviolet (UV) paint, a solvent-type paint or an aqueous paint, including synthetic resin particles 175. In this instance, the synthetic resin particles 175 in a type of beads may be formed from silica, glass, acrylic, urethane, or their copolymers or blends. Accordingly, because the transfer inducing material 170 includes the synthetic resin particles 175, printing of the design layer 110 on the release layer 120 is facilitated and the design layer 110 may be separated from the release layer 120 with ease and thus may be effectively transferred to the body. However, the transfer inducing material 170 including the synthetic resin particles 175 do not necessarily need to be formed in a form of coating between the design layer 110 and the release layer 120, and may be added to the design layer 110.

The protection film 130 is removed before the design layer 110 is transferred to the body, and is provided on the other surface (surface opposite to one surface) of the design layer 110. Here, the protection film 130 serves to protect the design layer 110. Also, a substance of the protection film 130 is not particularly limited, but the protection film 130 may be formed from a blend or a copolymer of at least one of PET, OPP, PP, PS, PE, PVC, PVDC, EVA, PU, TPU, polyamide, polyester, EVA, and acryl.

Meanwhile, the transferable make-up member 100 including the design layer 110, the release layer 120, and the like may be basically formed flat as shown in FIGS. 1 through 4, but is not necessarily limited thereto. That is, the transferable make-up member 100 is formed in three dimensions to have a curve so that the transferable make-up member 100 may be favorably transferred to the body of which a majority of parts have a curved shape, without wrinkling on the body. For example, as shown in FIG. 5, in the case where the transferable make-up member 100 is transferred around the eye rims 200, the transferable make-up member 100 is formed to have a curve corresponding to the curvature of the eye rims 200 and thus may be easily transferred.

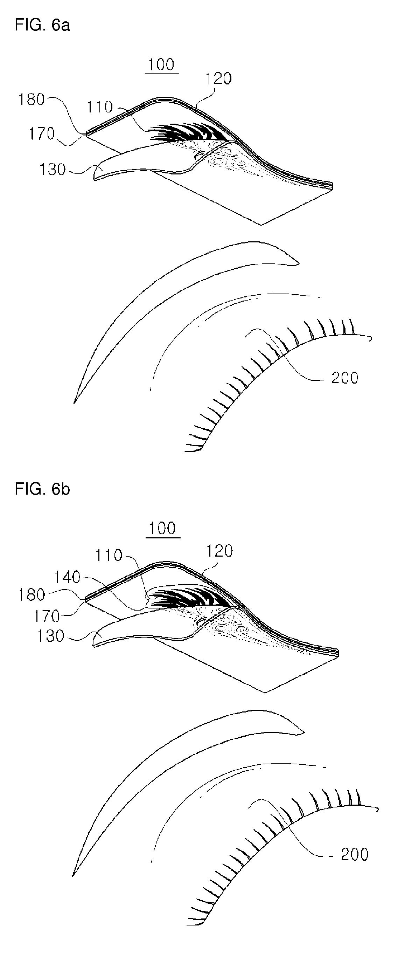

FIGS. 6 through 8 are perspective views illustrating a process of transferring a transferable make-up member according to an exemplary embodiment of the present disclosure, and with reference to this, a process of transferring the transferable make-up member according to this embodiment to the body 200 will be described.

First, as shown in FIGS. 6a and 6b, the protection film 130 is removed from the transferable make-up member 100. When the protection film 130 is removed, the design layer 110 with the adhesive substance 119 added thereto is exposed (see FIG. 6a). However, in case in which the separate adhesive layer 140 is provided, the adhesive layer 140 is exposed when the protection film 130 is removed (see FIG. 6b).

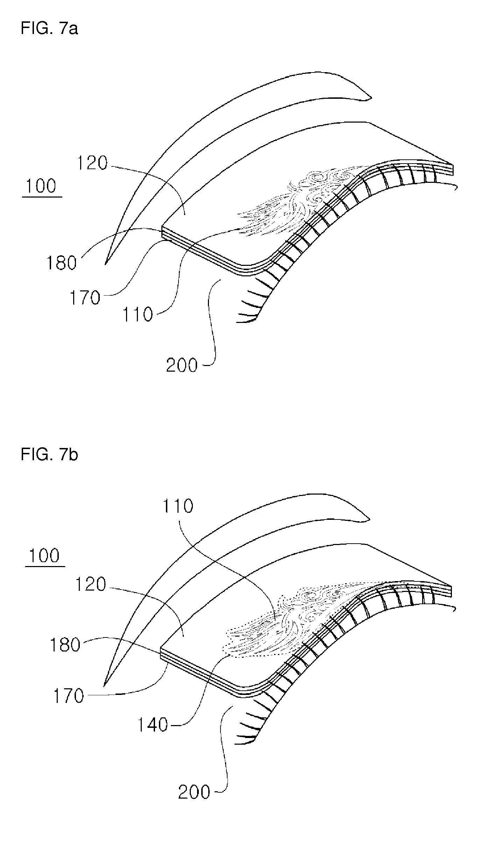

Subsequently, as shown in FIGS. 7a and 7b, the transferable make-up member 100 is closely attached to the body 200 (for example, eye rims). Because in the previous step, the design layer 110 with the adhesive substance 119 added thereto or the separate adhesive layer 140 is in an exposed state, the design layer 110 may be adhered to the body 200. In this instance, the release layer 120 may be transferred to the body 200 by a dry transfer technique or a water transfer technique.

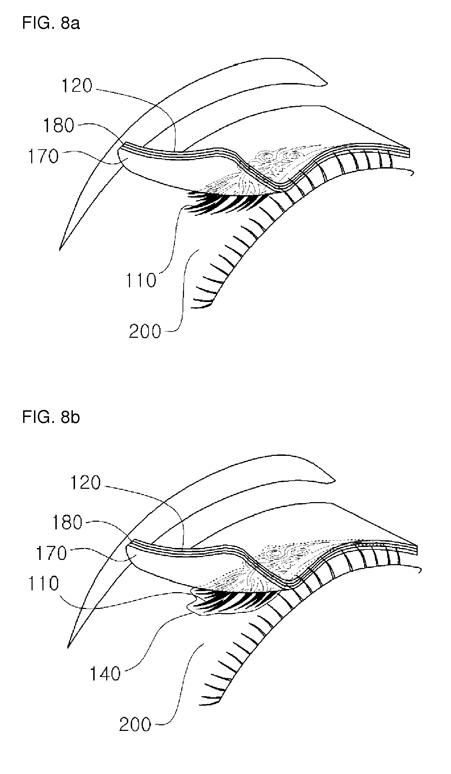

Subsequently, as shown in FIGS. 8a and 8b, the release layer 120 is removed from the transferable make-up member 100. Because in the previous step, the design layer 110 has already been adhered to the body 200, when the release layer 120 is separated from the design layer 110, the design layer 110 is finally exposed, and consequently, transferring is completed. In this instance, because the transfer inducing material 170 is coated between the design layer 110 and the release layer 120 or the transfer inducing material 170 is added to the design layer 110, the design layer 110 can be easily separated from the release layer 120 and thus may be effectively transferred to the body.

Although the description is provided based on a process of transferring eye line or eye shadow to assist the understanding of the present disclosure, the scope of protection is not limited thereto, and the design layer 110 may be transferred to any part of the body 200. For example, the design layer 110 may be transferred to a skin including eyes, lips, fingernails or toenails of the body 200. Besides, the design layer 110 may be transferred as Bindis or tattoos.

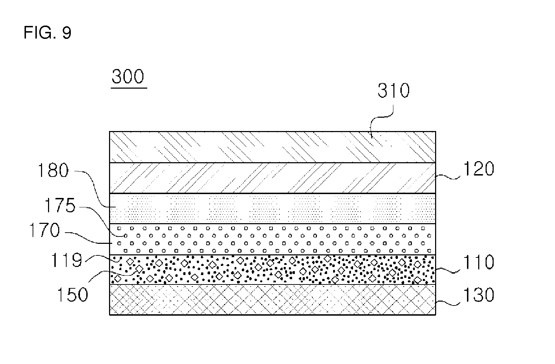

Meanwhile, FIG. 9 is a cross-sectional view illustrating a transferable make-up member according to another exemplary embodiment of the present disclosure. As shown in FIG. 9, the transferable make-up member 300 according to another exemplary embodiment of the present disclosure may further include an additional design layer 310, when compared to the transferable make-up member 100 according to the previous embodiment. Here, the additional design layer 310 may be printed and formed on the release layer 120. In this instance, the additional design layer 310 has a design presented thereon corresponding to the design layer 110. The additional design layer 310 may play a similar role to the first design layer 115 and the second design layer 117 described above. Specifically, in the case where the release layer 120 is formed from an opaque material, even though the design layer 110 includes the first and second design layers 115 and 117, a user may have difficulty in recognizing a design when transferring. Accordingly, by forming the additional design layer 310 on the exposed surface of the release layer 120, a user may recognize a design of the additional design layer 310 when transferring, and this is coincident with the design of the design layer 110 seen by others after transferring. In the end, as the additional design layer 310 is formed, inconsistency between a design recognized by a user when transferring and a design seen by others after transferring may be prevented.

While the present disclosure has been described in detail through the specific embodiments, this is just for specifying the present disclosure and the present disclosure is not limited in this regard, and it will be apparent to those skilled in the art that various changes and modifications may be made without departing from the spirit and scope of the disclosure.

Simple modifications and changes of the present disclosure fall within the domain of the present disclosure, and the specific scope of protection of the present disclosure will become apparent from the appended claims.

**Description of Numeric References:**

| | |
|---|---|
| 100, 300: Transferable make-up member | |
| 110: Design layer | |
| 113: Base layer | 115: First design layer |
| 117: Second design layer | 119: Adhesive substance |
| 120: Release layer | 120a: One end of release layer |
| 120b: The other end of release layer | 120c: Handle |
| 130: Protection film | 140: Adhesive layer |
| 150: Light reflective particle | 160: Cutting line |
| 170: Transfer inducing material | 175: Synthetic resin particle |
| 180: Elastic layer | 200: Body |
| 310: Additional design layer | |

## Claims

1. A transferable make-up member, comprising:
a design layer including an ink and a vehicle, the design layer having a design presented thereon to be transferred to a body;
a release layer provided on one surface of the design layer, the release layer which is removed after the design layer is transferred to the body;
a transfer inducing material coated between the design layer and the release layer or added to the design layer, the transfer inducing material including synthetic resin particles; and
a protection film provided on the other surface of the design layer, the protection film which is removed before the design layer is transferred to the body.

2. The transferable make-up member according to claim 1, wherein the design layer has an adhesive substance added thereto.

3. The transferable make-up member according to claim 1 or 2, further comprising:
an adhesive layer formed between the design layer and the protection film.

4. The transferable make-up member according to any of claims 1-3, wherein the transferable make-up member is formed flat or in three dimensions to have a curve.

5. The transferable make-up member according to any of claims 1-4, wherein the design layer is transferred to a skin including eyes, lips, fingernails or toenails of the body.

6. The transferable make-up member according to any of claims 1-5, wherein the design layer is formed by silk screen printing, offset printing, mold printing, or gravure printing.

7. The transferable make-up member according to any of claims 1-6, wherein the synthetic resin particles include silica, glass, acrylic, or urethane.

8. The transferable make-up member according to any of claims 1-7, wherein the transfer inducing material is formed from an ultraviolet (UV) paint, a solvent-type paint or an aqueous paint, including the synthetic resin particles.

9. The transferable make-up member according to any of claims 1-8, wherein the release layer includes a handle formed to protrusively extend outward from one end.

10. The transferable make-up member according to any of claims 1-9, wherein the design layer is formed in a multilayered structure, and comprises:
a base layer; and
a first design layer and a second design layer respectively formed on both surfaces of the base layer, and
the first design layer and the second design layer have designs presented thereon corresponding to each other; and
wherein the first design layer and the second design layer are preferably disposed at corresponding locations to each other.

11. The transferable make-up member according to any of claims 1-10, wherein the design layer has light reflective particles added thereto, including glitter, bling, spangle, confetti, or pearl.

12. The transferable make-up member according to any of claims 1-11, wherein the release layer is formed of a polyethyleneterephthalate (PET) film or a polyethylene (PE) film coated with a silicone release agent or a fluoride release agent; and
wherein the release layer preferably has an elastic layer formed from paper, polyethylene, ethylene vinyl acetate, polypropylene, latex, urethane, or foam.

13. The transferable make-up member according to any of claims 1-12, wherein the release layer is formed from paper, polyethylene, ethylene vinyl acetate, polypropylene, latex, urethane, or foam.

14. The transferable make-up member according to any of claims 1-13, wherein the release layer has a cutting line which is cut with respect to one point between both ends in a lengthwise direction.

15. The transferable make-up member according to any of claims 1-14, wherein the release layer includes an additional design layer having a design presented thereon corresponding to the design layer.
